# EUROPÄISCHE PATENTSCHRIFT

(11) **EP 2 211 829 B1**
(45) Veröffentlichungstag und Bekanntmachung des Hinweises auf die Patenterteilung: **27.04.2011**
(21) Anmeldenummer: 08805064.6
(22) Anmeldetag: 06.10.2008
(51) Int. Cl.: A61K 8/37, A61K 8/66, A61Q 13/00, C11D 3/386, C11D 3/50

(54) **VERBESSERUNG DER DUFTWIRKUNG VON RIECHSTOFF-ESTERN**
IMPROVEMENT OF THE FRAGRANCE EFFECT OF PERFUME ESTERS
AMÉLIORATION DE L'EFFET PARFUMANT D'ESTERS DE SUBSTANCES ODORANTES

(30) Priorität: 08.11.2007 DE 102007053615
(43) Veröffentlichungstag der Anmeldung: 04.08.2010
(73) Patentinhaber: Henkel AG & Co. KGaA, 40589 Düsseldorf (DE)
(72) Erfinder: BESSLER, Cornelius, 40231 Düsseldorf (DE); BAUER, Andreas, 41564 Kaarst (DE); LAHN, Wolfgang, 47877 Willich (DE); GERIGK, Andreas, 41812 Erkelenz (DE); WELLING, Hermann-Josef, 40789 Monheim (DE)
(86) Internationale Anmeldenummer: PCT/EP2008/063315
(87) Internationale Veröffentlichungsnummer: WO 2009/059858

(56) Entgegenhaltungen:
- EP-A- 0 142 886
- US-B1- 6 228 821

## Beschreibung

Die vorliegende Erfindung betrifft ein Verfahren zur Fixierung von Riechstoff-Estern auf harten und/oder weichen Oberflächen. Weiterhin betrifft die Erfindung die Verwendung von Hydrolasen (insbesondere Hemicellulase, Protease und/oder Amylase) zur Fixierung von Riechstoff-Estern auf harten und/oder weichen Oberflächen beim Waschen bzw. Reinigen. Außerdem betrifft die Erfindung die Verwendung von Hydrolasen (insbesondere Hemicellulase, Protease und/oder Amylase) in Riechstoff-Ester-haltigen Wasch- oder Reinigungsmitteln zur VerlängerungNerstärkung der Duftwirkung der Riechstoff-Ester.

Bei der Wäsche von Textilien beziehungsweise der Reinigung harter Oberflächen wie zum Beispiel Badezimmerfliesen erwartet man nicht nur eine optisch einwandfreie Sauberkeit, sondern auch das Fehlen von etwaigen unangenehmen Gerüchen auf den gereinigten Textilen beziehungsweise harten Oberflächen. Ein Zurückbleiben von Riechstoffen, die aus dem Reinigungs-, Wasch- oder Wäschenachbehandlungsmittel stammen und für einen Wohlgeruch sorgen, wird dabei als besonders angenehm empfunden und verstärkt den Sauberkeitseindruck. Zum Beispiel wird beim manuellen Waschen von Textilien, das normalerweise im Waschbecken durchgeführt wird, von vielen Anwendern der zurückbleibende Geruch im Becken sowie an den Händen als angenehm empfunden. Verbraucher wünschen sich für gewaschene Wäsche einen Duft, der nicht nur am Produkt selbst und direkt nach dem Waschen noch zu bemerken ist, sondern insbesondere auch über mehrere Tage, am besten sogar Wochen noch deutlich am behandelten Objekt wahrnehmbar ist. Allerdings ist die Menge an Parfüm, die aus dem Wasch- oder Spülvorgang aus wässriger Lösung z.B. auf Textilien aufzieht, oftmals zu gering, um auch über längere Zeit einen wahrnehmbaren Dufteindruck zu gewährleisten. Da Riechstoffe besonders kostenintensive Inhaltsstoffe von Wasch- und Reinigungsmitteln sind, ist man bestrebt, sie nur in geringen Mengen einzusetzen. Der Verlust an diesen Inhaltsstoffen (zum Beispiel in einer Waschmaschine) ist für die Hersteller und die Verbraucher solcher Mittel gleichermaßen unbefriedigend.

Überraschenderweise wurde nun gefunden, dass durch den Einsatz von bestimmten Enzymen, nämlich von Hydrolasen, vozugsweise ausgewählt aus a) Glycosidasen, dabei vorzugsweise a1) Hemicellulasen, besonders bevorzugt Mannanase und/oder, a2) Stärke abbauende Enzymen, besonders bevorzugt Amylase und/oder, b) Proteasen, vorzugsweise Subtilasen, insbesondere Subtilisine, die Haftung von Riechstoff-Estern an Oberflächen, wie zum Beispiel an Textilien, an harten Gegenständen oder am menschlichen Körper, verbessert werden kann, wenn man diese Enzyme zusammen mit Riechstoff-Ester(n) beim Waschen beziehungsweise Reinigen der Oberflächen verwendet.

Gegenstand der vorliegenden Erfindung ist vor diesem Hintergrund ein Verfahren zur Fixierung von Riechstoff-Estern auf harten und/oder weichen Oberflächen, wobei man die Oberfläche mit einer wässrigen Behandlungsflotte, umfassend Riechstoff-Ester und Hydrolase, über einen Zeitraum von 1 Minute bis 300 Minuten bei einer Temperatur unter 95°C behandelt. In einer besonders bevorzugten Ausführungsform der Erfindung ist die Hydrolase ausgewählt aus a) Glycosidasen, vorzugsweise
a1) Hemicellulasen, besonders bevorzugt Mannanase und/oder,
a2) Stärke abbauende Enzymen, besonders bevorzugt Amylase und/oder, b) Proteasen, vorzugsweise Subtilasen, insbesondere Subtilisine,

Besonders bevorzugt wird Mannanase, Protease und/oder Amylase eingesetzt.

Es konnte überraschend gefunden werden, dass bei dem genannten Verfahren zur Behandlung von harten und/oder weichen Oberflächen mit einer wässrigen Behandlungsflotte, welche Riechstoff-Ester und zuvor genanntes Enzym enthält, eine "Fixierung" im Sinne einer verbesserten Haftung der Riechstoff-Ester auf bzw. an den behandelten harten und/oder weichen Oberflächen erzielt wird, welche sich in einer Verlängerung und Verstärkung der Duftwirkung der Riechstoff-Ester insbesondere auf der trockenen Oberfläche manifestiert. D.h., der mit dem Riechstoff-Ester verbundene Duft ist nicht nur am Produkt selbst und direkt nach dem Waschen wahrnehmbar, sondern ist sogar noch über mehrere Tage, am besten sogar Wochen nach dem erfolgten Wasch- oder Reinigungsvorgang bzw. Behandlungsvorgang noch deutlich wahrnehmbar. Mit anderen Worten kann auf den behandelten Oberflächen über längere Zeit ein besser wahrnehmbarer Dufteindruck gewährleistet werden. Es konnte gefunden werden, dass die erfindungsgemäß behandelten Oberflächen, insbesondere im Fall von Textilien, nach der Behandlung und dabei vorzugsweise im trockenen Zustand, besonders intensiv dufteten, beispielsweise bei einer geruchlichen Bewertung am trockenen Objekt 7 Tage nach der erfolgten Behandlung. Dabei ist der verbesserte Dufteindruck auf den Riechstoff-Ester als solchen zurückzuführen, dessen Duft von den erfindungsgemäß behandelten Oberflächen ausgeht, wie olfaktorisch geschulte Experten bestätigen können, beispielsweise bei einer geruchlichen Bewertung am trockenen Objekt 7 Tage nach der erfolgten Behandlung. Es konnte aber auch gefunden werden, dass Riechstoffkompositionen (bzw. Parfümöle), also wohlriechende Gemische von zumindest zwei oder mehr Riechstoffen, in dem genannten Zusammenhang von der Anwesenheit der Riechstoff-Ester profitieren und zwar in einer Weise, dass auch die Duftwirkung der anderen Riechstoffe verlängert wird, so dass die Gesamtduftwirkung der betreffenden Riechstoff-Ester-haltigen Riechstoffkomposition verlängert wird. Diese allgemeine Duftverstärkung im Sinne einer verlängerten Gesamtduftwirkung, insbesondere am trockenen Objekt, ist jedoch an das Vorhandensein von Riechstoff-Ester in der betreffenden Riechstoffkomposition zwingend gebunden. Vorteilhafterweise sind Riechstoff-Ester in der gesamten Riechstoff-Komposition in Mengen von zumindest 1 Gew.-% enthalten, vorzugsweise zumindest 2 Gew.-% und insbesondere zumindest 10 Gew.-%, Gew.-% bezogen auf die Gesamtmenge der enthaltenen Riechstoffe. Diese Mindestgrenze kann auch höher liegen, z.B. bei zumindest 15 Gew.-%, 20 Gew.-%, 25 Gew.-%, 30 Gew.-%, 40 Gew.-% oder 50 Gew.-%, Gew.-% bezogen auf die Gesamtmenge der enthaltenen Riechstoffe. Diese Mindestgrenze kann auch noch höher liegen, z.B. bei zumindest 60 Gew.-%, 70 Gew.-%, 80 Gew.-% oder 90 Gew.-%, Gew.-% bezogen auf die Gesamtmenge der enthaltenen Riechstoffe.

Unter weichen Oberflächen sollen im Kontext dieser Erfindung sowohl menschliche Haut als auch Haar und insbesondere Textilien, auch unterschiedlicher Zusammensetzung, zum Beispiel aus Baumwolle, Wolle, Seide, Polyester, Polyamid, Viskose und Mischgewebe jeglicher Art, verstanden werden. Unter harten Oberflächen sollen hier übliche harte Oberflächen wie Glas, Metall, Porzellan, Keramik, Steingut verstanden werden, insbesondere aus dem Haushaltsbereich und Sanitärbereich, z.B. in Form von Geschirr, Töpfen, Tellern, Pfannen, Fußböden, Fenstern, Kacheln, Steinplatten usw.

Erfindungsgemäß bevorzugt einzusetzende Riechstoffverbindungen vom Typ der Riechstoff-Ester sind insbesondere 3a,4,5,6,7,7a-Hexahydro-4,7-methano-1H-indenylacetat, Tricyclo[5.2.1.02,6]dec-4-en-8-yl acetat, 3a,4,5,6,7,7a-Hexahydro-4,7-methanoinden-6-yl acetat, 2-tert-Butylcyclo-hexylacetat, cis-2-tert-Butylcyclohexylacetat, trans-2-tert-Butylcyclohexylacetat, 4-tert-Butylcyclohexylacetat, cis-4-tert-Butylcyclohexylacetat, trans-4-tert-Butylcyclohexylacetat, 3a,4,5,6,7,7a-Hexahydro-4,7-methano-1H-indenyl propionat, 3a,4,5,6,7,7a-Hexahydro-4,7-methano-1H-inden-6-yl propionat, 3a,4,5,6,7,7a-Hexahydro-4,7-methano-1H-inden-5-yl propionat, Benzylsalicylat, Cyclohexylsalicylat, Pentylsalicylat, 2-Methylbutylsalicylat, Isopentylsalicylat, Exo-1,7,7-trimethylbicyclo[2.2.1]hept-2-yl acetat, p-Menth-1-en-8-yl acetat, Terpineolacetat, Benzylacetat, Hexylsalicylat, alpha,alpha-Dimethytphenethylacetat, 1-Phenylethylacetat, Linalylacetat, Phenethylacetat, Hexylacetat, Citronellylacetat, Geranylacetat, Nerylacetat, Citronellylacetat, p-Menthan-8-yl acetat, alpha,alpha-4-Trimethyl-cyclohexylmethylacetat, Nopyl-acetat, Ethylacetoacetat, 3-Methyl-2-butenyl acetat, 2-Cyclohexylethylacetat, Isononylacetat, Isobutylsalicylat, [3R-(3.alpha.,3a.beta.,7.beta.,8a.alpha.)]-2,3,4,7,8,8a-Hexahydro-3,8,8-trimethyl-1H-3a,7-methanoazulene-6-methylacetat, Phenethylphenylacetat, (Z)-3-Hexenyl-salicylat, (Z)-Hex-3-enylacetat, 1,3-Dimethyl-3-phenylbutylacetat, Methylsalicylat, Isopentylacetat, Nerylacetat, Cinnamylacetat, Menthylacetat, 1,2,3,3a,4,5,6,8a-Octahydro-2-isopropylidene-4,8-dimethylazulen-6-yl acetat, Allylphenoxyacetat, Decahydro-2-naphthylacetat, Myrcenylacetat, Methylphenylacetat, Ethylsalicylat, 3(or 4)-(4-Methylpenten-3-yl)cyclohex-3-ene-1-methylacetat, 1-Methyl-1-((3S,8S)-1,2,3,4,5,6,7,8-octahydro-3,8-dimethy)azuten-5-yl)ethylacetat, Ethylacetat, Phenylsalicylat, Phenethylsalicylat, p-Tolylacetat, p-Tolylphenylacetat und/oder 1,1,5-Trimethylhepta-4,6-dienylacetat. Darunter sind insbesondere Hexylacetat, Phenethylacetat und/oder 1-Phenylethylacetat hervorzuheben.

Als weitere Riechstoffe können zusammen mit den Riechstoff-Estern z.B. einzelne oder auch mehrere andere Riechstoffverbindungen, z.B. die synthetischen Produkte vom Typ der Ether, Aldehyde, Ketone, Alkohole und Kohlenwasserstoffe verwendet werden. Zu den bevorzugt einsetzbaren Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den bevorzugt einsetzbaren Ketonen z.B. die Jonone, α-Isomethylionon und Methyl-cedrylketon, zu den bevorzugt einsetzbaren Alkoholen Anethol, Citronellol, Eugenol, Geraniol, Linalool, Phenylethylalkohol und Terpineol, zu den bevorzugt einsetzbaren Kohlenwasserstoffen gehören hauptsächlich die Terpene und Balsame. Es ist besonders bevorzugt, wenn Mischungen verschiedener Riechstoffe in Kombination mit Riechstoff-Ester(n) verwendet werden, so dass eine besonders ansprechende und individuell gestaltbare Duftnote erzeugt werden kann. Derartige Mischungen von Einzelsubstanzen gelten im Sinne der vorliegenden Anmeldung als Riechstoffe bzw. Parfümöle bzw. Riechstoffkompositionen, wobei sich, wie schon betont wurde, der erfindungsgemäße Effekt der Verstärkung der Duftfixierung des Riechstoff-Esters an der behandelten Oberfläche, insbesondere bezüglich des trockenen Objektes, positiv auf den Dufteindruck der gesamten Riechstoffkomposition auswirkt. Das Parfümöl kann auch natürliche Riechstoffgemische umfassen, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeeröl, Vetiveröl, Olibanumöl, Galbanumöl, Labdanumöl und Lavandinöl. Vorzugsweise können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl, Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat in einem erfindungsgemäßen Riechstoffgemisch, welches also zwingend Riechstoff-Ester umfasst, enthalten sein.

Die erfindungsgemäß einzusetzenden Enzyme (Hydrolasen, wie insbesondere Mannanase, Protease und/oder Amylase) können vorzugsweise auch an Trägerstoffen adsorbiert und/oder in Hüllsubstanzen eingebettet sein. Dies entspricht einer bevorzugten Ausführungsform der Erfindung. Auf diese Weise können sie, sofern das erforderlich ist, noch besser gegen eine vorzeitige Inaktivierung geschützt werden.

Erfindungsgemäß können bevorzugt Proteasen (vorzugsweise Subtilasen, insbesondere Subtilisine) zur Fixierung von Riechstoff-Estern auf harten und/oder weichen Oberflächen eingesetzt werden. Vorzugsweise enthält ein erfindungsgemäß einsetzbares Mittel Savinase® und/oder eine gegebenenfalls gentechnisch veränderte Protease aus Bacillus lentus. Am meisten bevorzugt sind Proteasen vom Subtilisin-Typ. Proteasen können bekanntlich als Eiweiß spaltende Enzyme in Wasch- und Reinigungsmitteln zur Entfernung entsprechender Eiweiß-haltiger Anschmutzungen eingesetzt werden. Der Einsatz von Proteasen (vorzugsweise Subtilasen, insbesondere Subtilisinen) in Wasch- oder Reinigungsmitteln oder Kosmetika zum Zweck der von uns gefundenen überraschenden Verbesserung des Dufteindrucks von Riechstoff-Estern war dagegen bisher völlig unbekannt. Zu den erfindungsgemäß bevorzugt einsetzbaren Proteasen gehört neben den aus verschiedenen Bacillus Spezies erhältlichen oder gentechnisch modifizierten Proteasen wie Alcalase®, Esperase®, Savinase®, Durazym® oder Everlase® die Protease aus Bacillus lentus (BLAP), die unter alkalischen Bedingungen stabil und aktiv ist; sie kann wie in der internationalen Patentanmeldung WO 91/02792 beschrieben in Bacillus lentus (DSM 5483) produziert werden oder auch durch Fermentation von Bacillus licheniformis erhalten werden, der mit einem Expressionsplasmid transformiert wurde, welches das Gen für BLAP unter der Kontrolle des Promotors aus Bacillus licheniformis ATCC 53926 trägt. Die Zusammensetzung wie auch die räumliche Struktur von BLAP ist bekannt (D.W. Godette et al., J. Mol. Biol. 228, 580-595,1992). Diese Protease ist durch die in der zitierten Literatur beschriebene Sequenz aus 269 Aminosäuren, ein rechnerisches Molekulargewicht von 26 823 Dalton und einen theoretischen isoetektrischen Punkt von 9,3 charakterisiert. Durch Mutation erhältliche Varianten dieser Bacillus lentus DSM 5483 Protease sind in der US-amerikanischen Patentschrift US 5 340 735 beschrieben. Unter diesen sind Proteaseenzyme bevorzugt, die insbesondere bei mehrfacher Waschbehandlung von Textilien aus proteinogenen Fasern, beispielsweise textilen Flächengebilden aus Naturseide oder Wolle, ohne Verlust an Reinigungsleistung eine besonders geringe Substanzschädigung beziehungsweise Zerstörung der Faserverbände bewirken. Zu den erfindungsgemäß besonders brauchbaren Proteasen zählen neben der natürlich vorkommenden Protease aus Bacillus lentus auch gentechnisch veränderte Proteasen des obengenannten BLAP-Typs, bei denen in Position 211 (BLAP-Zählung) die an dieser Stelle in der Wildtyp-Protease vorhandene Aminosäure Leucin (L im gebräuchlichen Ein-Buchstaben-Code) gegen Asparaginsäure (D) beziehungsweise Glutaminsäure (E) ausgetauscht ist (L211D beziehungsweise L211E). Diese können wie in der internationalen Patentanmeldung WO 95/23221 beschrieben hergestellt werden. Stattdessen oder zusätzlich können weitere Veränderungen gegenüber der ursprünglichen Bacillus lentus-Protease, wie beispielsweise mindestens einer der Aminosäureaustausche S3T, V41, R99G, R99A, R99S, A188P, V193M und/oder V199I, vorgenommen werden. Besonders bevorzugt ist der Einsatz einer Variante, bei welcher mindestens einer der Aminosäureaustausche S3T, V4I , A188P, V193M, V199I und/oder L211D vorgenommen wurde. Bei der vorstehend beschriebenen Proteasenomenklatur über den Austausch einzelner Aminosäuren ist zu beachten, dass die Numerierung der Aminosäurepositionen in der Zählweise der alkalischen Protease aus Bacillus lentus (BLAP) sich von der Zählweise des Subtilisin BPN' unterscheidet. Es sei an dieser Stelle kurz klargestellt, dass mit dem Begriff der "erfindungsgemäß einsetzbaren Mittel" ein direkter Bezug zu dem erfindungsgemäßen Verfahren und zu der weiter unten beschriebenen erfindungsgemäßen Verwendung gegeben ist, in dem Sinne, dass das erfindungsgemäß einsetzbare Mittel, z.B. ein Waschmittel, enthaltend Riechstoff-Ester und Hydrolase, z.B. Mannanase, in dem erfindungsgemäßen Verfahren zur Anwendung gelangt, beispielsweise dadurch, dass man das betreffende Mittel zur Bereitstellung der für das Verfahren notwendigen wässrigen Behandlungsflotte einsetzt. Erfindungsgemäß können bevorzugt Stärke abbauende Enzyme, insbesondere Amylasen zur Fixierung von Riechstoff-Estern auf harten und/oder weichen Oberflächen eingesetzt werden. Amylasen haben normalerweise die Aufgabe, die Entfernung stärkehaltiger Anschmutzungen durch die katalytische Hydrolyse des Stärke-Polysaccharids zu erleichtern. Im Rahmen der Erfindung können vorzugsweise sowohl Amylasen vom Typ α-Amylase aus *Bacillus licheniformis,* die z.B. unter dem Handelsnamen Termamyl® von der Firma Novozymes vertrieben wird, wie auch gentechnisch veränderte Amylasen, das heißt solche mit im Vergleich zu natürlich vorkommenden Amylasen mit Hilfe gentechnologischer Methoden veränderter Aminosäuresequenz, zum Einsatz kommen, wie sie zum Beispiel aus den internationalen Patentanmeldungen WO 94/18314 oder WO 95/21247 bekannt sind. Weitere Beispiele für erfindungsgemäß bevorzugt einsetzbare Amylasen sind neben der alpha-Amylase aus Bacillus licheniformis die alpha-Amylasen aus B. amyloliquefaciens, aus B. stearothermophilus, aus Aspergillus niger und A. oryzae sowie die für den Einsatz in Wasch- und Reinigungsmitteln verbesserten Weiterentwicklungen aller vorgenannten Amylasen. Desweiteren sind für diesen Zweck die alpha-Amylase aus Bacillus sp. A 7-7 (DSM 12368) und die Cyclodextrin-Glucanotransferase (CGTase) aus B. agaradherens (DSM 9948) hervorzuheben. Der Einsatz von Amylasen in Wasch- oder Reinigungsmitteln oder Kosmetika zum Zweck der von uns gefundenen überraschenden Verbesserung des Dufteindrucks von Riechstoff-Estern war bisher unbekannt. Weiterhin können insbesondere Enzyme erfindungsgemäß eingesetzt werden, die unter dem Begriff Hemicellulasen zusammengefaßt werden. Hierzu gehören z.B. Mannanasen, Xanthanlyasen, Pektinlyasen (=Pektinasen), Pektinesterasen, Pektatlyasen, Xyloglucanasen (=Xylanasen), Pullulanasen und ß-Glucanasen. Diesbezüglich geeignete Enzyme sind z.B. unter den Namen Gamanase® und Pektinex AR® von der Firma Novozymes, unter dem Namen Rohapec® B1 L von der Firma AB Enzymes und unter dem Namen Pyrolase® von der Firma Diversa Corp., San Diego, CA, USA erhältlich. Die aus Bacillus subtilis gewonnene ß-Glucanase ist unter dem Namen Cereflo® von der Firma Novozymes erhältlich. Erfindungsgemäß ganz besonders bevorzugte Hemicellulasen sind Mannanasen, z.B. jene, welche beispielsweise unter den Handelsnamen Mannaway® von dem Unternehmen Novozymes oder Purabrite® von dem Unternehmen Genencor vertrieben werden. Mannanase kann im Zusammenhang mit Wasch- oder Reinigungsmitteln normalerweise zur Entfernung mannanhaltiger Rückstände bei der Textilwäsche eingesetzt werden. Der Einsatz von Mannanasen in Wasch- oder Reinigungsmitteln oder Kosmetika zum Zweck der von uns gefundenen überraschenden Verbesserung des Dufteindrucks von Riechstoff-Estern war bisher allerdings unbekannt.

Nach einer bevorzugten Ausführungsform des erfindungsgemäßen Verfahrens liegt die Behandlungsdauer der Oberflächen mit der wässrigen Behandlungsflotte im Bereich von 2 Minuten bis 120 Minuten, insbesondere von 10 Minuten bis 80 Minuten. Wenn die Temperatur der wässrigen Behandlungsflotte im Bereich von 15 °C bis 90 °C, insbesondere von 20°C bis 60 °C liegt, so liegt ebenfalls eine bevorzugte Ausführungsform des erfindungsgemäßen Verfahrens vor. Dabei ist es insbesondere vorteilhaft, wenn die Temperatur der wässrigen Behandlungsflotte während der gesamten Behandlungszeit im Bereich von 20 °C bis 60 °C liegt. Ist dies gewährleistet, lassen sich besonders gute Duftergebnisse erzielen, insbesondere trockene Objekte betreffend. Es sei an dieser Stelle klargestellt, dass mit dem Begriff "trockener Objekte" solche Oberflächen gemeint sind, z.B. Oberflächen von Textilien, welche zuerst einem erfindungsgemäßen Verfahren unterworfen wurden und folglich mit einer erfindungsgemäßen wäßrigen Behandlungsflotte in Kontakt traten, also feucht wurden und hernach der Trocknung überlassen wurden, beispielsweise an der Raumluft, bzw. einer Trocknung unterworfen wurden, beispielsweise in einem Wäschetrockner.

Die Konzentration der erfindungsgemäß einzusetzenden Hydrolase in der wässrigen Behandlungsflotte liegt nach einer bevorzugten Ausführungsform des erfindungsgemäßen Verfahrens im Bereich von 0,0001 mg/I bis 0,25 g/I, insbesondere von 0,01 mg/l bis 15 mg/I. Die Konzentration der erfindungsgemäß einzusetzenden Riechstoff-Ester in der wässrigen Behandlungsflotte liegt nach einer bevorzugten Ausführungsform des erfindungsgemäßen Verfahrens im Bereich von 0,0001 g/l bis 0,25 g/I, insbesondere von 0,001 g/I bis 0,05 g/I. Wenn die Behandlungsflotte anionisches und/oder nichtionisches Tensid umfasst, so liegt ebenfalls eine bevorzugten Ausführungsform des erfindungsgemäßen Verfahrens vor. Überraschenderweise konnte insbesondere auch bei Anwesenheit von größeren Mengen anionischem und/oder nichtionischem Tensid in einem erfindungsgemäß einsetzbaren Mittel, z.b. in einer Gesamtmenge > 5 Gew.-%, > 10 Gew.-% oder sogar > 15 Gew.-%, Gew.-% bezogen auf das gesamte Mittel, der Effekt der erfindungsgemäßen Fixierung von Riechstoff-Estern bestätigt werden.

Ein weiterer Gegenstand der Erfindung liegt in der Verwendung von Hydrolasen (vorzugsweise ausgewählt aus a) Glycosidasen, vorzugsweise a1) Hemicellulasen, besonders bevorzugt Mannanase und/oder, a2) Stärke abbauende Enzymen, besonders bevorzugt Amylase und/oder, b) Proteasen, vorzugsweise Subtilasen, insbesondere Subtilisine) zur Fixierung von Riechstoff-Estern auf harten und/oder weichen Oberflächen beim Waschen bzw. Reinigen der harten und/oder weichen Oberflächen mit Riechstoff-Ester-haltigen Behandlungsmitteln. Die Fixierung im Sinne einer verbesserten Anhaftung führt, wie oben bereits erklärt, zu einer Verlängerung/Verstärkung der Duftwirkung des Riechstoff-Esters auf der behandelten Oberfläche, insbesondere am trockenen Objekt, und befördert auch die Duftwirkung anderer Riechstoffe, wenn eine Riechstoff-Ester-haltige Riechstoffkomposition zum Einsatz kommt, wiederum insbesondere am trockenen Objekt, beispielsweise bestätigbar durch eine geruchliche Beurteilung der behandelten Objekte, vorzugsweise durch Parfumeure, insbesondere 7 Tage nach Trocknung. Die eben genannte erfindungsgemäße Verwendung zur Fixierung von Riechstoff-Estern auf Oberflächen ist insbesondere beim Waschen von Textilien, vorzugsweise in einer automatischen Waschmaschine, besonders vorteilhaft. Ein weiterer Vorteil liegt darin, dass diese erfindungsgemäße Fixierung auch dann noch mit einer Verlängerung/Verstärkung der Duftwirkung des Riechstoff-Esters auf der behandelten Oberfläche einhergeht, wenn man das gewaschene Textilgut anschließend einer Textiltrocknung in einem automatischen Textiltrocknern unterwirft, insbesondere in einem Abluft- oder einem Kondensationstrockner. Dies ist ein besonderer Vorteil, denn oftmals ist es so, dass durch den Trocknungsakt in einem automatischen Textiltrockner der Wohlgeruch des zu trocknenden Textils in sehr weitgehender Weise verlorengeht.

Wird bei der erfindungemäßen Verwendung die Hydrolase (vorzugsweise ausgewählt aus a) Glycosidasen, vorzugsweise a1) Hemicellulasen, besonders bevorzugt Mannanase und/oder, a2) Stärke abbauende Enzymen, besonders bevorzugt Amylase und/oder, b) Proteasen, vorzugsweise Subtilasen, insbesondere Subtilisine) in Gewichtsmengen, bezogen auf den Riechstoff-Ester, im Bereich von 1: 0,05 bis 1:20 eingesetzt, so handelt es sich um eine bevorzugte Ausführungsform der Erfindung.

Die Verwendung von Hydrolasen (vorzugsweise ausgewählt aus a) Glycosidasen, vorzugsweise a1) Hemicellulasen, besonders bevorzugt Mannanase und/oder, a2) Stärke abbauende Enzymen, besonders bevorzugt Amylase und/oder, b) Proteasen, vorzugsweise Subtilasen, insbesondere Subtilisine) in Riechstoff-Ester-haltigen, vorzugsweise flüssigen oder festen, Wasch- oder Reinigungsmitteln zur Verlängerung/Verstärkung der Duftwirkung der Riechstoff-Ester nach der Wasch- oder Reinigungsanwendung auf einer zu behandelnden harten und/oder weichen Oberfläche, insbesondere bezogen auf das trockene Objekt, ist ein weiterer Gegenstand der vorliegenden Erfindung. Die beschriebene Verlängerung/Verstärkung der Duftwirkung der Riechstoff-Ester ist auf deren verbesserter Fixierung auf der behandelten Oberfläche zurückzuführen. Durch die verbesserte Fixierung wird die Verflüchtigung des Riechstoff-Esters verlangsamt. Verbesserte Fixierung heißt nicht, dass die Abscheidung der Riechstoff-Ester auf den Oberflächen verbessert wird, sondern dass die abgeschiedenen Riechstoffe besser haften.

Es ist besonders bevorzugt, wenn das betreffende Wasch- oder Reinigungsmittel die Hydrolase (vorzugsweise ausgewählt aus a) Glycosidasen, vorzugsweise a1) Hemicellulasen, besonders bevorzugt Mannanase und/oder, a2) Stärke abbauende Enzymen, besonders bevorzugt Amylase und/oder, b) Proteasen, vorzugsweise Subtilasen, insbesondere Subtilisine) in Mengen von 0,0000001-5 Gew.-%, vorzugsweise 0,000001-4 Gew.-%, vorteilhafterweise 0,00001-3 Gew.-% , in weiter vorteilhafter Weise 0,0001- 2 Gew.-%, weiter vorteilhaft 0,001 -1 Gew.-%, noch weiter vorteilhaft 0,01-0,5 Gew.-%, insbesondere 0,05-0,1 Gew.-% enthält, Gew.-% bezogen auf das gesamte Mittel. Dabei können die genannten Ober- und Untergrenzen variiert werden, so dass die Hydrolase beispielweise in Mengen von 0,00001 bis 0,1 Gew.-% in dem betreffenden Wasch- oder Reinigungsmittel enthalten sein kann, z.B. in Mengen von 0,00001 bis 0,072 Gew.-%, Gew.-% bezogen auf das gesamte Mittel.

Bei der erfindungsgemäßen Verwendung kommen insbesondere Riechstoff-Ester ausgewählt aus 3a,4,5,6,7,7a-Hexahydro-4,7-methano-1H-indenylacetat, Tricyclo[5.2.1.02,6]dec-4-en-8-yl acetat, 3a,4,5,6,7,7a-Hexahydro-4,7-methanoinden-6-yl acetat, 2-tert-Butylcyclohexylacetat, cis-2-tert-Butylcyclohexylacetat, trans-2-tert-Butylcyclohexylacetat, 4-tert-Butylcyclohexylacetat, cis-4-tert-Butylcyclohexylacetat, trans-4-tert-Butylcyclohexylacetat, 3a,4,5,6,7,7a-Hexahydro-4,7-methano-1H-indenylpropionat, 3a,4,5,6,7,7a-Hexahydro-4,7-methano-1H-inden-6-yl propionat, 3a,4,5,6,7,7a-Hexahydro-4,7-methano-1H-inden-5-yl propionat, Benzylsalicylat, Cyclohexylsalicylat, Pentylsalicylat, 2-Methylbutylsalicylat, Isopentylsalicylat, Exo-1,7,7-trimethyl-bicyclo[2.2.1]hept-2-yl acetat, p-Menth-1-en-8-yl acetat, Terpineolacetat, Benzylacetat, Hexylsalicylat, alpha,alpha.-Dimethylphenethylacetat, 1-Phenylethylacetat, Linalylacetat, Phenethylacetat, Hexylacetat, Citronellylacetat, Geranylacetat, Nerylacetat, Citronellylacetat, p-Menthan-8-yl acetat, alpha.,.alpha.,4-Trimethylcyclohexylmethylacetat, Nopyl-acetat, Ethylacetoacetat, 3-Methyl-2-butenyl acetat, 2-Cyclohexylethylacetat, [3R-(3.alpha.,3a.beta.,7.beta.,8a.alpha.)]-2,3,4,7,8,8a-Hexahydro-3,8,8-trimethyl-1H-3a,7-methano-azulene-6-methylacetat, Phenethylphenylacetat, (Z)-3-Hexenylsalicylat, Isononylacetat, Isobutylsalicylat, (Z)-Hex-3-enylacetat, 1,3-Dimethyl-3-phenylbutyl acetat, Methyl salicylat, Isopentylacetat, Nerylacetat, Cinnamylacetat, Menthylacetat, 1,2,3,3a,4,5,6,8a-Octahydro-2-isopropylidene-4,8-dimethylazulen-6-yl acetat, Allyl phenoxyacetat, Decahydro-2-naphthylacetat, Myrcenylacetat, Methylphenylacetat, Ethylsalicylat, 3(or 4)-(4-Methylpenten-3-yl)cyclohex-3-ene-1-methylacetat, 1-Methyl-1-((3S,8S)-1,2,3,4,5,6,7,8-octahydro-3,8-dimethylazulen-5-yl)ethylacetat, Ethylacetat, Phenylsalicylat, Phenethylsalicylat, p-Tolylacetat, p-Tolylphenylacetat und/oder 1,1,5-Trimethylhepta-4,6-dienylacetat, insbesondere aber Hexylacetat, Phenethylacetat und/oder 1-Phenylethylacetat zum Einsatz.

Wie bereits klargestellt wurde, kann das erfindungsgemäße Verfahren unter Anwendung eines typischen Mittels, welches jedoch die Riechstoff-Ester und die Hydrolase enthalten muß, durchgeführt werden. Gleiches gilt für die erfindungsgemäße Verwendung. Bei einem solchen typischen Mittel, welches Riechstoff-Ester und Hydrolase enthält, kann es sich insbesondere um ein Textilwaschmittel beziehungsweise Textilpflegemittel, welches in teilchenförmiger oder flüssiger Form vorliegen kann, ein in entsprechender Form vorliegendes Reinigungsmittel für harte Oberflächen, zum Beispiel einen Fliesen-, einen Bad- oder Sanitärreiniger, oder ein Reinigungsmittel für den menschlichen Körper, zum Beispiel ein Haarshampoo, eine Reinigungslotion, ein Duschgel oder eine Stückseife, handeln. Insbesondere ist die erfindungsgemäße Lehre auch im Körperpflegebereich einsetzbar.

Zusätzlich können erfindungsgemäß einsetzbare Mittel, die als insbesondere pulverförmige Feststoffe, in nachverdichteter Teilchenform, in Formkörperform (insbesondere Tablettenform), als homogene Lösungen oder Suspensionen vorliegen können, alle üblichen Inhaltsstoffe aufweisen, die für den Einsatz des entsprechenden Mittels prädestiniert sind.

Erfindungsgemäß einsetzbare Wasch- oder Reinigungsmittel können insbesondere Buildersubstanzen, oberflächenaktive Tenside, ggf. weitere Enzyme, organische und/oder anorganische Persauerstoffverbindungen, Persauerstoff-Aktivatoren, wassermischbare organische Lösungsmittel, Sequestrierungsmittel, Elektrolyte, pH-Regulatoren, Verdicker und weitere Hilfsstoffe, wie soil release-Wirkstoffe, optische Aufheller, Vergrauungsinhibitoren, Farbübertragungsinhibitoren, Schaumregulatoren sowie Farbstoffe enthalten.

Die erfindungsgemäß einsetzbaren Mittel wie insbesondere Wasch- oder Reinigungsmittel können vorzugsweise Tenside enthalten, wobei insbesondere anionische Tenside, nichtionische Tenside und deren Gemische, aber auch kationische Tenside in Frage kommen. Geeignete nicht-ionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar. Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen. Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R¹ bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden beispielsweise mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyl-dimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N (2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Unter Esterquats sollen hier Verbindungen der allgemeinen Formel I, 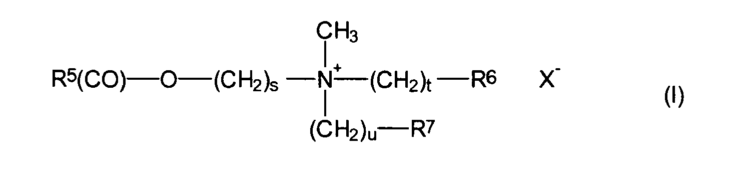 verstanden werden, in der R⁵ für einen Alkyl- oder Alkenylrest mit 12 bis 22 Kohlenstoffatomen und 0, 1, 2 oder 3 Doppelbindungen, R⁶ und R⁷ unabhängig voneinander für H, OH oder O(CO)R⁵, s, t und u jeweils unabhängig voneinander für den Wert 1, 2 oder 3 und X⁻ für ein Anion, insbesondere Halogenid, Methosulfat, Methophosphat oder Phosphat sowie Mischungen aus diesen, steht. Bevorzugt sind Verbindungen, die für R⁶ die Gruppe O(CO)R⁵ und für R⁵ einen Alkylrest mit 16 bis 18 Kohlenstoffatomen enthalten. Besonders bevorzugt sind Verbindungen, bei denen R⁷ zudem für OH steht. Beispiele für Verbindungen der Formel (V) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethylyN-(2-hydroxyethyl)ammonium-methosulfat. Werden quarternierte Verbindungen der Formel (V) eingesetzt, die ungesättigte Gruppen aufweisen, sind die Acylgruppen bevorzugt, deren korrespondierende Fettsäuren eine Jodzahl zwischen 5 und 80, vorzugsweise zwischen 10 und 60 und insbesondere zwischen 15 und 45 aufweisen und/oder die ein cis/trans-Isomerenverhältnis (in Mol-%) von größer als 30 : 70, vorzugsweise größer als 50 : 50 und insbesondere größer als 70 : 30 haben. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma Cognis Deutschland GmbH beziehungsweise die unter der Bezeichnung Rewoquat^{®} bekannten Produkte des Herstellers Goldschmidt-Witco.

Tenside sind in den erfindungsgemäß einsetzbaren Mitteln, wie insbesondere Wasch- oder Reinigungsmitteln, vorteilhafterweise enthalten und zwar in Mengenanteilen von vorzugsweise 5 Gew.-% bis 50 Gew.-%, insbesondere von 8 Gew.-% bis 30 Gew.-%. Insbesondere in erfindungsgemäß einsetzbaren Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäß einsetzbares Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamin-tetrakis(methylenphosphonsäure) und 1-Hydroxyethan-1,1-diphosphonsäure, polymere Hydroxyverbindungen wie Dextrin sowie polymere (Poly-)carbonsäuren, insbesondere die durch Oxidation von Polysacchariden zugänglichen Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative Molekülmasse zwischen 1 000 und 200 000, vorzugsweise zwischen 200 und 50 000 und insbesondere zwischen 3 000 und 10 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt. Derartige organische Buildersubstanzen können gewünschtenfalls in Mengen bis zu 40 Gew.-%, insbesondere bis zu 25 Gew.-%, vorzugsweise von 1 Gew.-% bis 8 Gew.-% in erfindungsgemäß einsetzbaren Mitteln enthalten sein. Mengen nahe der genannten Obergrenze werden vorzugsweise in pastenförmigen oder flüssigen, insbesondere wasserhaltigen, erfindungsgemäß einsetzbaren Mitteln eingesetzt. Erfindungsgemäß einsetzbare Wäschenachbehandlungsmittel können gegebenenfalls auch frei von organischem Builder sein.

Als wasserlösliche anorganische Buildermaterialien kommen für die erfindungsgemäß einsetzbaren Mittel insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den üblichen Methoden bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäß einsetzbaren Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäß einsetzbaren Mitteln eingesetzt werden. Bei einer weiteren bevorzugten Ausführungsform erfindungsgemäß einsetzbaren Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen können in den erfindungsgemäß einsetzbaren Mitteln, wie insbesondere Waschmitteln vorzugsweise in Mengen bis zu 60 Gew.-%, insbesondere von 5 Gew.-% bis 40 Gew.-%, enthalten sein. Erfindungsgemäß einsetzbare Wäschenachbehandlungsmittel sind vorzugsweise frei von anorganischem Builder.

Als geeignete Persauerstoffverbindungen kommen in den erfindungsgemäß einsetzbaren Mitteln insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein erfindungsgemäßes Waschmittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), sowie acylierte Phenolsulfonate, insbesondere n-Nonanoyl- oder Isononanoyloxybenzolsulfonat (n- bzw. iso-NOBS). Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf das gesamte einzusetzende Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren in den erfindungsgemäß einsetzbaren Mitteln enthalten sein. Zu den in Frage kommenden Übergangsmetallverbindungen gehören insbesondere Mangan-, Eisen-, Cobalt-, Ruthenium- oder Molybdän-Salenkomplexe und deren N-Analogverbindungen, Mangan-, Eisen-, Cobalt-, Ruthenium- oder Molybdän-Carbonylkomplexe, Mangan-, Eisen-, Cobalt-, Ruthenium-, Molybdän-, Titan-, Vanadium- und Kupfer-Komplexe mit stickstoffhaltigen Tripod-Liganden, Cobalt-, Eisen-, Kupfer- und Ruthenium-Amminkomplexe. Kombinationen aus Bleichaktivatoren und Übergangsmetall-Bleichkatalysatoren können ebenfalls eingesetzt werden. Bleichverstärkende Übergangsmetallkomplexe, insbesondere mit den Zentralatomen Mn, Fe, Co, Cu, Mo, V, Ti und/oder Ru, können in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt werden.

Als in den erfindungsgemäß einsetzbaren Mitteln zusätzlich verwendbaren Enzymen kommen solche aus der Klasse der Cutinasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Diese eben genannten, optional einsetzbaren Enzyme können an Trägerstoffen adsorbiert und/oder in Hüllsubstanzen eingebettet sein, um sie gegen vorzeitige Inaktivierung zu schützen.

Die erfindungsgemäß einsetzbaren Mittel können als optische Aufheller beispielsweise Derivate der Diaminostilbendisulfonsäure beziehungsweise deren Alkalimetallsalze enthalten. Geeignet sind zum Beispiel auch Salze der 4,4'-Bis(2-anilino-4-morpholino-1,3,5-triazinyl-6-amino)stilben-2,2'-disulfonsäure usw.

Zu den geeigneten Schauminhibitoren gehören beispielsweise Organopolysiloxane und deren Gemische mit mikrofeiner, gegebenenfalls silanierter Kieselsäure sowie Pa-raffinwachse und deren Gemische mit silanierter Kieselsäure oder Bisfettsäure-alkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die erfindungsgemäß einsetzbaren Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Waschmittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die erfindungsgemäß einsetzbaren Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone mit Molgewichten von 15 000 bis 50 000 wie auch Polyvinylpyrrolidone mit Molgewichten über 1 000 000, insbesondere von 1 500 000 bis 4 000 000, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist zum Einsatz in erfindungsgemäßen Mitteln vorzugsweise eine durchschnittliche Molmasse im Bereich von 10 000 bis 60 000, insbesondere im Bereich von 25 000 bis 50 000 auf. Unter den Copolymeren sind solche aus Vinylpyrrolidon und Vinylimidazol im Molverhältnis 5:1 bis 1:1 mit einer durchschnittlichen Molmasse im Bereich von 5 000 bis 50 000, insbesondere 10 000 bis 20 000 bevorzugt.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäß einsetzbaren Mittel, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel können in den erfindungsgemäß einsetzbaren Mitteln, wie vorzugsweise Wasch- oder Reinigungsmitteln, vorzugsweise in Mengen von nicht über 30 Gew.-%, insbesondere von 6 Gew.-% bis 20 Gew.-%, vorhanden sein.

Zur Einstellung eines gewünschten, sich durch die Mischung der übrigen Komponenten nicht von selbst ergebenden pH-Werts können die erfindungsgemäß einsetzbaren Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren können in den erfindungsgemäß einsetzbaren Mitteln vorzugsweise nicht über 20 Gew.-%, insbesondere von 1,2 Gew.-% bis 17 Gew.-%, enthalten sein.

Die Herstellung fester erfindungsgemäß einsetzbarer Mittel bereitet keine Schwierigkeiten und kann in im Prinzip bekannter Weise, zum Beispiel durch Sprühtrocknen oder Granulation, erfolgen, wobei z.B. Persauerstoffverbindung und Bleichkatalysator, sofern enthalten, gegebenenfalls später zugesetzt werden. Zur Herstellung erfindungsgemäß einsetzbaren Mittel mit erhöhtem Schüttgewicht, insbesondere im Bereich von 650 g/l bis 950 g/l, ist ein einen Extrusionsschritt aufweisendes Verfahren bevorzugt.

Gemäß einer weiteren bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder überhaupt nicht verwenden können. In diesem Zusammenhang sind beispielsweise Handwaschmittel zu nennen.

### Beispiele

| | |
|---|---|
| Waschbedingungen | |
| Waschgerät: | Miele® Novotronic® W 308 |
| Waschprogramm: | Einlaugenverfahren Normalprogramm Koch-Buntwäsche |
| Waschtemperatur: | 40 °C |
| Flottenvolumen: | 17 I |
| Wasserhärte: | 16 °dH |
| Füllwäsche: | 3,5 kg saubere Wäsche |
| Gewebe: | Frotteehandtücher, 100% Baumwolle |

**Tabelle 1: Eingesetzte Waschmittelzusammensetzungen (Inhaltsstoffangaben in Gew.-%)**

| | W1 | V1 |
|---|---|---|
| Fettalkoholethersulfat | 5 | 5 |
| Fettalkohol C_{12/14} 7 EO | 12 | 12 |
| C₁₂₋₁₄-Alkylpolyglykosid | 2 | 2 |
| Fettsäure C₁₂₋₁₈ | 5 | 5 |
| Glycerin | 5 | 5 |
| Na-Citrat | 1 | 1 |
| Na-Polyacrylat | 0,2 | 0,2 |
| Hexylacetat | 0,1 | 0,1 |
| Mannanase | 0,01 | - |
| Wasser | auf 100 | |

Die Textilien wurden 3 Mal mit einem Waschmittel W1 der oben angegebenen Zusammensetzung, welches 0,01 Gew.-% Mannanase enthielt, unter den oben angegebenen Bedingungen gewaschen und nach der letzten Wäsche an der Raumluft getrocknet. Parallel dazu wurden unter den gleichen Bedingungen Textilien mit dem Waschmittel V1, das heißt unter Abwesenheit der Mannanase, behandelt. Die Textilien wurden 6 ausgebildeten und erfahrenen Parfumeuren vorgelegt und von diesen geruchlich vergleichend beurteilt. Der Dufteindruck wurde sowohl für die feuchte Wäsche als auch für trockene Wäsche nach 7 Tagen Lagerung in einem offenen Regal bewertet.

Während der Dufteindruck an feuchter Wäsche vergleichbar war, zeigte sich beim Einsatz des erfindungsgemäßen Mittels W1 an der trockenen Wäsche 7 Tagen Lagerung in einem offenen Regal ein merklich intensiverer Duft als bei Einsatz von V1. Dieser Test wurde in völlig analoger Weise noch ein weiteres Mal durchgeführt und führte zu dem gleichen Ergebnis.

Weiter wurden die Waschmittel X1 und Z1 gegen V1 getestet und zwar unter den gleichen Bedingungen wie zuvor beschrieben. X1 unterschied sich von V1 nur dadurch, dass es 0,01 Gew.-% Protease enthielt. Z1 unterscheid sich von V1 nur dadurch, dass es 0,01 Gew.-% Amylase enthielt. Beim Einsatz des erfindungsgemäßen Mittels X1 zeigte sich an der trockenen Wäsche nach 7 Tagen Lagerung in einem offenen Regal ein merklich intensiverer Duft als bei Einsatz von V1. Der Dufteindruck an feuchter Wäsche war vergleichbar. Beim Einsatz des erfindungsgemäßen Mittels Z1 zeigte sich an der trockenen Wäsche nach 7 Tagen Lagerung in einem offenen Regal ebenfalls ein merklich intensiverer Duft als bei Einsatz von V1. Der Dufteindruck an feuchter Wäsche war vergleichbar.

## Patentansprüche

1. Verfahren zur Fixierung von Riechstoff-Estern auf harten und/oder weichen Oberflächen, wobei man die Oberfläche mit einer wässrigen Behandlungsflotte, umfassend Riechstoff-Ester und Hydrolase, über einen Zeitraum von 1 Minute bis 300 Minuten bei einer Temperatur unter 95°C behandelt.

2. Verfahren nach Anspruch 1, **dadurch gekennzeichnet, dass** die Hydrolase ausgewählt ist aus
a) Glycosidasen, vorzugsweise
a1) Hemicellulasen, besonders bevorzugt Mannanase und/oder,
a2) Stärke abbauende Enzymen, besonders bevorzugt Amylase und/oder,
b) Proteasen, vorzugsweise Subtilasen, insbesondere Subtilisine.

3. Verfahren nach Anspruch 1 oder 2, **dadurch gekennzeichnet, dass** die Behandlungsdauer im Bereich von 2 Minuten bis 120 Minuten, insbesondere von 10 Minuten bis 80 Minuten liegt.

4. Verfahren nach einem der Ansprüche 1 bis 3, **dadurch gekennzeichnet, dass** die Temperatur der wässrigen Behandlungsflotte im Bereich von 15 °C bis 90 °C, insbesondere von 20°C bis 60 °C liegt.

5. Verfahren nach einem der Ansprüche 1 bis 4, **dadurch gekennzeichnet, dass** die Konzentration der Hydrolase, vorzugsweise ausgewählt aus
Glycosidase und/oder Protease, in der wässrigen Behandlungsflotte im Bereich von 0,0001 mg/l bis 0,25 g/l, insbesondere von 0,01 mg/l bis 15 mg/l liegt.

6. Verfahren nach einem der Ansprüche 1 bis 5, **dadurch gekennzeichnet, dass** die Konzentration an Riechstoff-Ester in der wässrigen Behandlungsflotte im Bereich von 0,0001 g/l bis 0,25 g/l, insbesondere von 0,001 g/l bis 0,05 g/l liegt.

7. Verfahren nach einem der Ansprüche 1 bis 6, **dadurch gekennzeichnet, dass** die Behandlungsflotte anionisches und/oder nichtionisches Tensid umfasst.

8. Verfahren nach einem der Ansprüche 1 bis 7, **dadurch gekennzeichnet, dass** der Riechstoff-Ester ausgewählt ist aus 3a,4,5,6,7,7a-Hexahydro-4,7-methano-1H-indenylacetat, Tricyclo-[5.2.1.02,6]dec-4-en-8-yl acetat, 3a,4,5,6,7,7a-Hexahydro-4,7-methanoinden-6-yl acetat, 2-tert-Butylcyclohexylacetat, cis-2-tert-Butylcyclohexylacetat, trans-2-tert-Butylcyclohexylacetat, 4-tert-Butylcyclohexylacetat, cis-4-tert-Butylcyclohexylacetat, trans-4-tert-Butylcyclohexylacetat, 3a,4,5,6,7,7a-Hexahydro-4,7-methano-1H-indenyl propionat, 3a,4,5,6,7,7a-Hexahydro-4,7-methano-1H-inden-6-yl propionat, 3a,4,5,6,7,7a-Hexahydro-4,7-methano-1H-inden-5-yl propionat, Benzylsalicylat, Cyclohexylsalicylat, Pentylsalicylat, 2-Methylbutylsalicylat, Isopentylsalicylat, Exo-1,7,7-trimethylbicyclo[2.2.1]hept-2-yl acetat, p-Menth-1-en-8-yl acetat, Terpineolacetat, Benzylacetat, Hexylsalicylat, alpha,alpha.-Dimethylphenethylacetat, 1-Phenylethylacetat, Linalylacetat, Phenethylacetat, Hexylacetat, Citronellylacetat, Geranylacetat, Nerylacetat, Citronellylacetat, p-Menthan-8-yl acetat, alpha,alpha-4-Trimethylcyclohexylmethylacetat, Nopyl-acetat, Ethylacetoacetat, 3-Methyl-2-butenyl acetat, 2-Cyclohexylethylacetat, [3R-(3.alpha.,3a.beta.,7.beta.,8a.alpha.)]-2,3,4,7,8,8a-Hexahydro-3,8,8-trimethyl-1 H-3a,7-methano-azulene-6-methylacetat, Phenethylphenylacetat, (Z)-3-Hexenylsalicylat, Isononylacetat, Isobutylsalicylat, (Z)-Hex-3-enylacetat, 1,3-Dimethyl-3-phenylbutyl acetat, Methylsalicylat, Isopentylacetat, Nerylacetat, Cinnamylacetat, Menthylacetat, 1,2,3,3a,4,5,6,8a-Octahydro-2-isopropylidene-4,8-dimethylazulen-6-yl acetat, Allylphenoxyacetat, Decahydro-2-naphthylacetat, Myrcenylacetat, Methylphenylacetat, Ethylsalicylat, 3(or 4)-(4-Methylpenten-3-yl)cyclohex-3-ene-1-methylacetat, 1-Methyl-1-((3S,8Sr1,2,3,4,5,6,7,8-octahydro-3,8-dimethylazulen-5-yl)ethylacetat, Ethylacetat, Phenylsalicylat, Phenethylsalicylat, p-Tolylacetat, p-Tolylphenytacetat und/oder 1,1,5-Trimethylhepta-4,6-dienylacetat, insbesondere aber Hexylacetat, Phenethylacetat und/oder 1-Phenylethylacetat enthält.

9. Verwendung von Hydrolasen zur Fixierung von Riechstoff-Estern auf harten und/oder weichen Oberflächen beim Waschen bzw. Reinigen der harten und/oder weichen Oberflächen mit Riechstoff-Ester-haltigen Behandlungsmitteln, wobei die Hydrolase vorzugsweise ausgewählt ist aus
a) Glycosidasen, vorzugsweise
a1) Hemicellulasen, besonders bevorzugt Mannanase und/oder,
a2) Stärke abbauende Enzymen, besonders bevorzugt Amylase und/oder,
b) Proteasen, vorzugsweise Subtilasen, insbesondere Subtilisine.

10. Verwendung nach Anspruch 9 zur Verlängerung/Verstärkung der Duftwirkung von Riechstoff-Estern beim Waschen von Textilien, vorzugsweise in einer automatischen Waschmaschine.

11. Verwendung nach Anspruch 9 oder 10, **dadurch gekennzeichnet, dass** man die Hydrolase in Gewichtsmengen, bezogen auf den Riechstoff-Ester, im Bereich von 1 : 0,05 bis 1 : 20 einsetzt.

12. Verwendung von Hydrolasen, vorzugsweise ausgewählt aus
a) Glycosidasen, vorzugsweise
a1) Hemicellulasen, besonders bevorzugt Mannanase und/oder,
a2) Stärke abbauende Enzymen, besonders bevorzugt Amylase und/oder,
b) Proteasen, vorzugsweise Subtilasen, insbesondere Subtilisine in Riechstoff-Ester-haltigen Wasch- oder Reinigungsmitteln zur Verlängerung/Verstärkung der Duftwirkung der Riechstoff-Ester auf einer zu behandelnden harten und/oder weichen Oberfläche nach der Wasch- oder Reinigungsanwendung.

13. Verwendung nach Anspruch 12 in flüssigen oder festen Wasch- oder Reinigungsmitteln.

14. Verwendung nach Anspruch 12 oder 13, **dadurch gekennzeichnet, dass** das Wasch- oder Reinigungsmittel Hydrolase, vorzugsweise ausgewählt aus
a) Glycosidasen, vorzugsweise
a1) Hemicellulasen, besonders bevorzugt Mannanase und/oder,
a2) Stärke abbauende Enzymen, besonders bevorzugt Amylase und/oder,
b) Proteasen, vorzugsweise Subtilasen, insbesondere Subtilisine in Mengen von 0,0000001-5 Gew.-%, vorzugsweise 0,000001-4 Gew.-%, vorteilhafterweise 0,00001-3 Gew.-% , in weiter vorteilhafter Weise 0,0001- 2 Gew.-%, weiter vorteilhaft 0,001 -1 Gew.-%, noch weiter vorteilhaft 0,01-0,5 Gew.-%, insbesondere 0,05-0,1 Gew.-% enthält, Gew.-% bezogen auf das gesamte Mittel.

15. Verwendung nach einem der Ansprüche 9-14, wobei Riechstoff-Ester ausgewählt aus 3a,4,5,6,7,7a-Hexahydro-4,7-methano-1 H-indenylacetat, Tricyclo[5.2.1.02,6]dec-4-en-8-yl acetat, 3a,4,5,6,7,7a-Hexahydro-4,7-methanoinden-6-yl acetat, 2-tert-Butylcyclohexylacetat, cis-2-tert-Butylcyclohexylacetat, trans-2-tert-Butylcyclohexylacetat, 4-tert-Butylcyclohexylacetat, cis-4-tert-Butylcyclohexylacetat, trans-4-tert-Butylcyclohexylacetat, 3a,4,5,6,7,7a-Hexahydro-4,7-methano-1 H-indenylpropionat, 3a,4,5,6,7,7a-Hexahydro-4,7-methano-1H-inden-6-yl propionat, 3a,4,5,6,7,7a-Hexahydro-4,7-methano-1H-inden-5-yl propionat, Benzylsalicylat, Cyclohexylsalicylat, Pentylsalicylat, 2-Methylbutylsalicylat, Isopentylsalicylat, Exo-1,7,7-trimethyl-bicyclo[2.2.1]hept-2-yl acetat, p-Menth-1-en-8-yl acetat, Terpineolacetat, Benzylacetat, Hexylsalicylat, alpha,alpha.-Dimethylphenethylacetat, 1-Phenylethylacetat, Linalylacetat, Phenethylacetat, Hexylacetat, Citronellylacetat, Geranylacetat, Nerylacetat, Citronellylacetat, p-Menthan-8-yl acetat, .alpha.,.alpha.,4-Trimethylcyclohexylmethylacetat, Nopyl-acetat, Ethylacetoacetat, 3-Methyl-2-butenyl acetat, 2-Cyclohexylethylacetat, [3R-(3.alpha.,3a.beta.,7.beta.,8a.alpha.)]-2,3,4,7,8,8a-Hexahydro-3,8,8-trimethyl-1H-3a,7-methano-azulene-6-methylacetat, Phenethylphenylacetat, (Z)-3-Hexenylsalicylat, Isononylacetat, Isobutylsalicylat, (Z)-Hex-3-enylacetat, 1,3-Dimethyl-3-phenylbutyl acetat, Methyl salicylat, Isopentylacetat, Nerylacetat, Cinnamylacetat, Menthylacetat, 1,2,3,3a,4,5,6,8a-0ctahydro-2-isopropylidene-4,8-dimethylazulen-6-yl acetat, Allyl phenoxyacetat, Decahydro-2-naphthylacetat, Myrcenylacetat, Methylphenylacetat, Ethylsalicylat, 3(or 4)-(4-Methy)penten-3-y))cyc)ohex-3-ene-1-methylacetat, 1-Methyl-1-((3S,8S)-1 ,2,3,4,5,6,7 ,8-octahydro-3,8-dimethylazulen-5-yl)ethylacetat, Ethylacetat, Phenylsalicylat, Phenethylsalicylat, p-Tolylacetat, p-Tolylphenylacetat und/oder 1,1,5-Trimethylhepta-4,6-dienylacetat, insbesondere aber Hexylacetat, Phenethylacetat und/oder 1-Phenylethylacetat zum Einsatz kommt.

## Claims

1. Process for fixing perfume esters to hard and/or soft surfaces, wherein the surface is treated at a temperature below 95 °C for a period of 1 minute to 300 minutes with an aqueous treatment liquor comprising perfume ester and hydrolase.

2. Process according to claim 1 wherein the hydrolase is selected from
a) glycosidases, preferably
a1) hemicellulases, particularly preferably mannanase and/or,
a2) starch-degrading enzymes, particularly preferably amylase and/or,
b) proteases, preferably subtilases, especially subtilisins.

3. Process according to claim 1 or 2 wherein the treatment time is in the range of 2 to 120 minutes, especially from 10 to 80 minutes.

4. Process according to one of claims 1 to 3 wherein the temperature of the aqueous treatment liquor is in the range 15°C to 90 °C, especially from 20 °C to 60 °C.

5. Process according to one of claims 1 to 4 wherein the concentration of the hydrolase, preferably selected from glycosidase and/or protease, in the aqueous treatment liquor is in the range 0.0001 mg/l to 0.25 g/l, especially from 0.01 mg/l to 15 mg/l.

6. Process according to one of claims 1 to 5 wherein the concentration of perfume ester in the aqueous treatment liquor is in the range 0.0001 g/l to 0.25 g/l, especially from 0.001 g/l to 0.05 g/l.

7. Process according to one of claims 1 to 6 wherein the treatment liquor contains anionic and/or non-ionic surfactant.

8. Process according to one of claims 1 to 7 wherein the perfume ester is selected from 3a,4,5,6,7,7a-hexahydro-4,7-methano-1H-indenyl acetate, tricyclo-[5.2.1.02,6]dec-4-en-8-yl acetate, 3a,4,5,6,7,7a-hexahydro-4,7-methanoinden-6-yl acetate, 2-tert-butylcyclohexyl acetate, cis-2-tert-butylcyclohexyl acetate, trans-2-tert-butylcyclohexyl acetate, 4-tert-butylcyclohexyl acetate, cis-4-tert-butylcyclohexyl acetate, trans-4-tert-butylcyclohexyl acetate, 3a,4,5,6,7,7a-hexahydro-4,7-methano-1H-indenyl propionate, 3a,4,5,6,7,7a-hexahydro-4,7-methano-1H-inden-6-yl propionate, 3a,4,5,6,7,7a-hexahydro-4,7-methano-1H-inden-5-yl propionate, benzyl salicylate, cyclohexyl salicylate, pentyl salicylate, 2-methylbutyl salicylate, isopentyl salicylate, exo-1,7,7-trimethylbicyclo[2.2.1]hept-2-yl acetate, p-menth-l-en-8-yl acetate, terpineol acetate, benzyl acetate, hexyl salicylate, alpha, alpha.-dimethylphenethyl acetate, 1-phenylethyl acetate, linalyl acetate, phenethyl acetate, hexyl acetate, citronellyl acetate, geranyl acetate, neryl acetate, citronellyl acetate, p-menthan-8-yl acetate, alpha,alpha-4-trimethylcyclohexylmethyl acetate, nopyl acetate, ethyl acetoacetate, 3-methyl-2-butenyl acetate, 2-cyclohexylethyl acetate, [3R-(3.alpha.,3a.beta.,7.beta.,8a.alpha.)]-2,3,4,7,8,8a-hexahydro-3,8,8-trimethyl-1H-3a,7-methano-azulene-6-methyl acetate, phenethylphenyl acetate, (Z)-3-hexenyl salicylate, isononyl acetate, isobutyl salicylate, (Z)-hex-3-enyl acetate, 1,3-dimethyl-3-phenylbutyl acetate, methyl salicylate, isopentyl acetate, neryl acetate, cinnamyl acetate, menthyl acetate, 1,2,3,3a,4,5,6,8a-octahydro-2-isopropylidene-4,8-dimethylazulen-6-yl acetate, allylphenoxy acetate, decahydro-2-naphthyl acetate, myrcenyl acetate, methylphenyl acetate, ethyl salicylate, 3(or 4)-(4-methylpenten-3-yl)cyclohex-3-ene-1-methyl acetate, 1-methyl-1-((3S,8S)-1,2,3,4,5,6,7,8-octahydro-3,8-dimethylazulen-5-yl)ethyl acetate, ethyl acetate, phenyl salicylate, phenethyl salicylate, p-tolyl acetate, p-tolylphenyl acetate and/or 1,1,5-trimethylhepta-4,6-dienyl acetate, but in particular comprises hexyl acetate, phenethyl acetate and/or 1-phenylethyl acetate.

9. Use of hydrolases for fixing perfume esters to hard and/or soft surfaces when washing or cleaning the hard and/or soft surfaces with perfume ester-containing treatment agents, wherein the hydrolase is preferably selected from
a) glycosidases, preferably
a1) hemicellulases, particularly preferably mannanase and/or,
a2) starch-degrading enzymes, particularly preferably amylase and/or,
b) proteases, preferably subtilases, especially subtilisins.

10. Use according to claim 9 for prolonging/strengthening the fragrance effect of perfume esters when washing textiles, preferably in an automatic washing machine.

11. Use according to claim 9 or 10 wherein the hydrolase is employed, based on the perfume ester, in the range of 1: 0.05 to 1: 20 by weight.

12. Use of hydrolases, preferably selected from
a) glycosidases, preferably
a1) hemicellulases, particularly preferably mannanase and/or,
a2) starch-degrading enzymes, particularly preferably amylase and/or,
b) proteases, preferably subtilases, especially subtilisins in perfume ester-containing washing or cleaning agents for prolonging/strengthening the fragrance effect of the perfume esters on a treated hard and/or soft surface after the washing or cleaning application.

13. Use according to claim 12 in liquid or solid washing or cleaning agents.

14. Use according to claim 12 or 13 wherein the washing or cleaning agent comprises hydrolase, preferably selected from
a) glycosidases, preferably
a1) hemicellulases, particularly preferably mannanase and/or,
a2) starch-degrading enzymes, particularly preferably amylase and/or,
b) proteases, preferably subtilases, especially subtilisins in amounts of 0.0000001-5 wt.%, preferably 0.000001-4 wt.%, more preferably 0.00001-3 wt.%, in a further preferred manner 0.0001-2 wt.%, further advantageously 0.001-1 wt.%, even further advantageously 0.01-0.5 wt.%, especially 0.05-0.1 wt.%, the wt.% being based on the total agent.

15. Use according to one of claims 9-14 wherein the perfume ester selected from 3a,4,5,6,7,7a-hexahydro-4,7-methano-1H-indenyl acetate, tricyclo-[5.2.1.02,6]dec-4-en-8-yl acetate, 3a,4,5,6,7,7a-hexahydro-4,7-methanoinden-6-yl acetate, 2-tert-butylcyclohexyl acetate, cis-2-tert-butylcyclohexyl acetate, trans-2-tert-butylcyclohexyl acetate, 4-tert-butylcyclohexyl acetate, cis-4-tert-butylcyclohexyl acetate, trans-4-tert-butylcyclohexyl acetate, 3a,4,5,6,7,7a-hexahydro-4,7-methano-1H-indenyl propionate, 3a,4,5,6,7,7a-hexahydro-4,7-methano-1H-inden-6-yl propionate, 3a,4,5,6,7,7a-hexahydro-4,7-methano-1H-inden-5-yl propionate, benzyl salicylate, cyclohexyl salicylate, pentyl salicylate, 2-methylbutyl salicylate, isopentyl salicylate, exo-1,7,7-trimethylbicyclo[2.2.1]hept-2-yl acetate, p-menth-l-en-8-yl acetate, terpineol acetate, benzyl acetate, hexyl salicylate, alpha, alpha.-dimethylphenethyl acetate, 1-phenylethyl acetate, linalyl acetate, phenethyl acetate, hexyl acetate, citronellyl acetate, geranyl acetate, neryl acetate, citronellyl acetate, p-menthan-8-yl acetate, alpha,alpha-4-trimethylcyclohexylmethyl acetate, nopyl acetate, ethyl acetoacetate, 3-methyl-2-butenyl acetate, 2-cyclohexylethyl acetate, [3R-(3.alpha.,3a.beta.,7.beta.,8a.alpha.)]-2,3,4,7,8,8a-hexahydro-3,8,8-trimethyl-1H-3a,7-methano-azulene-6-methyl acetate, phenethylphenyl acetate, (Z)-3-hexenyl salicylate, isononyl acetate, isobutyl salicylate, (Z)-hex-3-enyl acetate, 1,3-dimethyl-3-phenylbutyl acetate, methyl salicylate, isopentyl acetate, neryl acetate, cinnamyl acetate, menthyl acetate, 1,2,3,3a,4,5,6,8a-octahydro-2-isopropylidene-4,8-dimethylazulen-6-yl acetate, allylphenoxy acetate, decahydro-2-naphthyl acetate, myrcenyl acetate, methylphenyl acetate, ethyl salicylate, 3(or 4)-(4-methylpenten-3-yl)cyclohex-3-ene-1-methyl acetate, 1-methyl-1-((3S,8S)-1,2,3,4,5,6,7,8-octahydro-3,8-dimethylazulen-5-yl)ethyl acetate, ethyl acetate, phenyl salicylate, phenethyl salicylate, p-tolyl acetate, p-tolylphenyl acetate and/or 1,1,5-trimethylhepta-4,6-dienyl acetate, but in particular hexyl acetate, phenethyl acetate and/or 1-phenylethyl acetate is used.

## Revendications

1. Procédé pour la fixation d'esters de parfums sur des surfaces dures et/ou souples, dans lequel on traite la surface avec un bain de traitement aqueux comprenant des esters de parfums et de l'hydrolase pendant un laps de temps de 1 minute à 300 minutes à une température inférieure à 95 °C.

2. Procédé selon la revendication 1, **caractérisé en ce que** l'hydrolase est choisie parmi
a) des glycosidases, de préférence
a1) des hémicellulases, de manière particulièrement préférée la mannanase et/ou
a2) des enzymes de décomposition de l'amidon, de manière particulièrement préférée l'amylase et/ou
b) des protéases, de préférence des subtilases, en particulier des subtilisines.

3. Procédé selon la revendication 1 ou 2, **caractérisé en ce que** le temps de traitement se situe dans la plage de 2 minutes à 120 minutes, en particulier de 10 minutes à 80 minutes.

4. Procédé selon l'une quelconque des revendications 1 à 3, **caractérisé en ce que** la température du bain de traitement aqueux se situe dans la plage de 15 °C à 90 °C, en particulier de 20 °C à 60 °C.

5. Procédé selon l'une quelconque des revendications 1 à 4, **caractérisé en ce que** la concentration de l'hydrolase, de préférence choisie parmi des glycosidases et/ou des protéases, dans le bain de traitement aqueux, se situe dans la plage de 0,0001 mg/l à 0,25 g/l, en particulier de 0,01 mg/l à 15 mg/l.

6. Procédé selon l'une quelconque des revendications 1 à 5, **caractérisé en ce que** la concentration des esters de parfums dans le bain de traitement aqueux, se situe dans la plage de 0,0001 g/l à 0,25 g/l, en particulier de 0,001 g/l à 0,05 g/l.

7. Procédé selon l'une quelconque des revendications 1 à 6, **caractérisé en ce que** le bain de traitement comprend un agent tensioactif anionique et/ou non ionique.

8. Procédé selon l'une quelconque des revendications 1 à 7, **caractérisé en ce que** l'ester de parfum est choisi parmi l'acétate de 3a,4,5,6,7,7a-hexahydro-4,7-méthano-1H-indényle, l'acétate de tricyclo[5.2.1.02,6]déc-4-én-8-yle, l'acétate de 3a,4,5,6,7,7a-hexahydro-4,7-méthano-indén-6-yle, l'acétate de 2-tert-butylcyclohexyle, l'acétate de cis-2-tert-butylcyclohexyle, l'acétate de trans-2-tert-butylcyclohexyle, l'acétate de 4-tert-butylcyclohexyle, l'acétate de cis-4-tert-butylcyclohexyle, l'acétate de trans-4-tert-butylcyclohexyle, le propionate de 3a,4,5,6,7,7a-hexahydro-4,7-méthano-1H-indényle, le propionate de 3a,4,5,6,7,7a-hexahydro-4,7-méthano-1H-indén-6-yle, le propionate de 3a,4,5,6,7,7a-hexahydro-4,7-méthano-1H-indén-5-yle, le salicylate de benzyle, le salicylate de cyclohexyle, le salicylate de pentyle, le salicylate de 2-méthylbutyle, le salicylate d'isopentyle, le salicylate d'exo-1,7,7-triméthylbicyclo-[2.2.1]hept-2-yle, l'acétate de p-menth-1-én-8-yle, l'acétate de terpinéol, l'acétate de benzyle, le salicylate d'hexyle, l'acétate de α,α-diméthylphénéthyle, l'acétate de 1-phényléthyle, l'acétate de linalyle, l'acétate de phénéthyle, l'acétate d'hexyle, l'acétate de citronnellyle, l'acétate de géranyle, l'acétate de néryle, l'acétate de citronnellyle, l'acétate de p-menthan-8-yle, l'acétate de α,α-4-triméthylcyclohexylméthyle, l'acétate de nopyle, l'acéto-acétate d'éthyle, l'acétate de 3-méthyl-2-butényle, l'acétate de 2-cyclohexyléthyle, l'acétate de [3R-(3α,3aβ,7β,8aα)]-2,3,4,7,8,8a-hexahydro-3,8,8-triméthyl-1H-3a,7-méthano-azulène-6-méthyle, l'acétate de phénéthylphényle, le salicylate de (Z)-3-hexényle, l'acétate d'isononyle, le salicylate d'isobutyle, l'acétate de (Z)-hex-3-ényle, l'acétate de 1,3-diméthyl-3-phénylbutyle, le salicylate de méthyle l'acétate d'isopentyle, l'acétate de néryle, l'acétate de cinnamyle, l'acétate de menthyle, l'acétate de 1,2,3,3a,4,5,6,8a-octahydro-2-isopropylidène-4,8-diméthylazulén-6-yle, le phénoxyacétate d'allyle, l'acétate de décahydro-2-naphtyle, l'acétate de myrcényle, l'acétate de méthylphényle, le salicylate d'éthyle, l'acétate de 3(ou 4)-(4-méthylpentén-3-yl)cyclohex-3-ène-1-méthyle, l'acétate de 1-méthyl-1-((3S,8S)-1,2,3,4,5,6,7,8-octahydro-3,8-diméthylazulén-5-yl)éthyle, l'acétate d'éthyle, le salicylate de phényle, le salicylate de phénéthyle, l'acétate de p-tolyle, l'acétate de p-tolylphényle, et/ou l'acétate de 1,1,5-triméthylhepta-4,6-diényle, mais contient en particulier de l'acétate d'hexyle, de l'acétate de phénéthyle et/ou de l'acétate de 1-phényléthyle.

9. Utilisation d'hydrolases pour la fixation d'esters de parfums sur des surfaces dures et/ou souples lors du lavage respectivement du nettoyage des surfaces dures et/ou souples avec des agents de traitement contenant des esters de parfums, l'hydrolase étant choisie de préférence parmi :
a) des glycosidases, de préférence
a1) des hémicellulases, de manière particulièrement préférée la mannanase et/ou
a2) des enzymes de décomposition de l'amidon, de manière particulièrement préférée l'amylase et/ou
b) des protéases, de préférence des subtilases, en particulier des subtilisines.

10. Utilisation selon la revendication 9, pour la prolongation/le renforcement de l'effet parfumant d'esters de parfums lors du lavage des textiles, de préférence dans un lave-linge automatique.

11. Utilisation selon la revendication 9 ou 10, **caractérisée en ce qu'**on met en oeuvre l'hydrolase dans des proportions, rapportées à l'ester de parfum, dans la plage de 1 : 0,05 à 1 : 20.

12. Utilisation d'hydrolases, de préférence choisies parmi
a) des glycosidases, de préférence
a1) des hémicellulases, de manière particulièrement préférée la mannanase et/ou
a2) des enzymes de décomposition de l'amidon, de manière particulièrement préférée l'amylase et/ou
b) des protéases, de préférence des subtilases, en particulier des subtilisines
dans des agents de lavage ou de nettoyage contenant des esters de parfums pour la prolongation/le renforcement de l'effet parfumant des esters de parfums sur une surface dure et/ou souple à traiter, après l'application du lavage ou du nettoyage.

13. Utilisation selon la revendication 12, dans des agents de lavage ou de nettoyage liquides ou solides.

14. Utilisation selon la revendication 12 ou 13, **caractérise en ce que** l'agent de lavage ou de nettoyage contient l'hydrolase, de préférence choisies parmi
a) des glycosidases, de préférence
a1) des hémicellulases, de manière particulièrement préférée la mannanase et/ou
a2) des enzymes de décomposition de l'amidon, de manière particulièrement préférée l'amylase et/ou
b) des protéases, de préférence des subtilases, en particulier des subtilisines
dans des quantités de 0,0000001 à 5 % en poids, de préférence de 0,000001 à 4 % en poids, de préférence de 0,00001 à 3 % en poids, de manière plus préférée de 0,0001 à 2 % en poids, de manière davantage préférée de 0,001 à 1 % en poids, de manière encore plus préférée de 0,01 à 0,5 % en poids, en particulier de 0,05 à 0,1 % en poids, les % en poids se rapportant à l'agent dans sa totalité.

15. Utilisation selon l'une quelconque des revendications 9 à 14, dans laquelle on met en oeuvre des esters de parfums choisis parmi l'acétate de 3a,4,5,6,7,7a-hexahydro-4,7-méthano-1H-indényle, l'acétate de tricyclo[5.2.1.02,6]déc-4-én-8-yle, l'acétate de 3a,4,5,6,7,7a-hexahydro-4,7-méthano-indén-6-yle, l'acétate de 2-tert-butylcyclohexyle, l'acétate de cis-2-tert-butylcyclohexyle, l'acétate de trans-2-tert-butylcyclohexyle, l'acétate de 4-tert-butylcyclohexyle, l'acétate de cis-4-tert-butylcyclohexyle, l'acétate de trans-4-tert-butylcyclohexyle, le propionate de 3a,4,5,6,7,7a-hexahydro-4,7-méthano-1H-indényle, le propionate de 3a,4,5,6,7,7a-hexahydro-4,7-méthano-1H-indén-6-yle, le propionate de 3a,4,5,6,7,7a-hexahydro-4,7-méthano-1H-indén-5-yle, le salicylate de benzyle, le salicylate de cyclohexyle, le salicylate de pentyle, le salicylate de 2-méthylbutyle, le salicylate d'isopentyle, le salicylate d'exo-1,7,7-triméthylbicyclo-[2.2.1]hept-2-yle, l'acétate de p-menth-1-én-8-yle, l'acétate de terpinéol, l'acétate de benzyle, le salicylate d'hexyle, l'acétate de α,α-diméthylphénéthyle, l'acétate de 1-phényléthyle, l'acétate de linalyle, l'acétate de phénéthyle, l'acétate d'hexyle, l'acétate de citronnellyle, l'acétate de géranyle, l'acétate de néryle, l'acétate de citronnellyle, l'acétate de p-menthan-8-yle, l'acétate de α,α-4-triméthylcyclohexylméthyle, l'acétate de nopyle, l'acéto-acétate d'éthyle, l'acétate de 3-méthyl-2-butényle, l'acétate de 2-cyclohexyléthyle, l'acétate de [3R-(3α,3αβ,7β,8aα)]-2,3,4,7,8,8a-hexahydro-3,8,8-triméthyl-1 H-3a,7-méthano-azulène-6-méthyle, l'acétate de phénéthylphényle, le salicylate de (Z)-3-hexényle, l'acétate d'isononyle, le salicylate d'isobutyle, l'acétate de (Z)-hex-3-ényle, l'acétate de 1,3-diméthyl-3-phénylbutyle, le salicylate de méthyle, l'acétate d'isopentyle, l'acétate de néryle, l'acétate de cinnamyle, l'acétate de menthyle, l'acétate de 1,2,3,3a,4,5,6,8a-octahydro-2-isopropylidène-4,8-diméthylazulén-6-yle, le phénoxyacétate d'allyle, l'acétate de décahydro-2-naphtyle, l'acétate de myrcényle, l'acétate de méthylphényle, le salicylate d'éthyle, l'acétate de 3(ou 4)-(4-méthylpentén-3-yl)cyclohex-3-ène-1-méthyle, l'acétate de 1-méthyl-1-((3S,8S)-1,2,3,4,5,6,7,8-octahydro-3,8-diméthylazulén-5-yl)éthyle, l'acétate d'éthyle, le salicylate de phényle, le salicylate de phénéthyle, l'acétate de p-tolyle, l'acétate de p-tolylphényle, et/ou l'acétate de 1,1,5-triméthylhepta-4,6-diényle, mais en particulier l'acétate d'hexyle, l'acétate de phénéthyle et/ou l'acétate de 1-phényléthyle.
